# EUROPEAN PATENT SPECIFICATION

(11) **EP 3 233 774 B1**
(45) Date of publication and mention of the grant of the patent: **30.01.2019**
(21) Application number: 15813374.4
(22) Date of filing: 16.12.2015
(51) Int. Cl.: C07C 29/17, C07C 31/12, C07C 31/20, C07D 307/08

(54) **PROCESS FOR THE PRODUCTION OF N-BUTANOL AND 1,4-BUTANEDIOL FROM FURAN**
VERFAHREN ZUR HERSTELLUNG VON N-BUTANOL UND 1,4-BUTANDIOL AUS FURAN
PROCÉDÉ POUR LA PRODUCTION DE N-BUTANOL ET 1,4-BUTANÉDIOL À PARTIR DE FURANE

(30) Priority: 18.12.2014 EP 14199023
(43) Date of publication of application: 25.10.2017
(73) Proprietor: Shell Internationale Research Maatschappij B.V., 2596 HR The Hague (NL)
(72) Inventor: LANGE, Jean Paul Andre Marie Joseph Gishlain, 1031 HW Amsterdam (NL); WADMAN, Sipke Hidde, 1031 HW Amsterdam (NL)
(74) Representative: Shell Legal Services IP
(86) International application number: PCT/EP2015/080105
(87) International publication number: WO 2016/097062

(56) References cited:
- US-A- 4 476 332

## Description

### Field of the Invention

The present invention relates to a process for the production of n-butanol and 1,4-butanediol from furan.

### Background of the Invention

Furan and its derivatives are useful precursors for industrial chemicals. Furan may be converted into n-butanol, 1,4-butanediol (1,4-BDO) and tetrahydrofuran (THF), all of which are valuable chemicals used industrially. In particular, n-butanol is used as a solvent in paints, coatings, varnishes, resins, dyes, as an extraction agent in the production of antibiotics, hormones and vitamins, and as a precursor of certain polymers. It is also a precursor and/or a component of certain pharmaceuticals, herbicides and cosmetics. 1,4-BDO is used as an industrial solvent and in the manufacture of some types of polymers and polyurethanes. THF, on the other hand, is used in the production of elastic fibres such as elastane/spandex (marketed as e.g. Lycra®), as well as an industrial solvent for PCV and in varnishes.

Although n-butanol and 1,4-BDO can be derived from furan, they are usually produced industrially from petrochemical (fossil fuel) derived feedstocks such as acetylene and propylene, and not from furan, via a number of routes.

One industrial route for the production of n-butanol uses propylene, which is hydroformylated in the presence of a rhodium-based homogeneous catalyst to butyraldehyde. This step is followed by the hydrogenation of butyraldehyde to n-butanol.

An industrial route for the production of 1,4-BDO requires the reaction of acetylene with two equivalents of formaldehyde followed by hydrogenation of the resultant 1,4-butynediol to form 1,4-butanediol. In an alternative process, propylene oxide is converted to allyl alcohol. The allyl alcohol is then hydroformylated to form 4-hydroxybutyraldehyde, which may be hydrogenated to form 1,4-butanediol. Although it co-produces THF, a route that is becoming more popular for the production of 1,4-BDO is the oxidation of butane to maleic anhydride followed its selective hydrogenation to 1,4-BDO, as well as THF. Other traditional routes use butadiene, allyl acetate or succinic acid as starting materials.

One industrial route for the production of THF usually proceeds via the hydrogenation of furan over a nickel catalyst (McKillip, W.J., ACS Symposium Series, Issue 385, 1989, Pages 408-423). In turn, Furan may be produced by Paal-Knorr synthesis which reacts 1,4-diketones with phosphorus pentoxide (P₂O₅), or by Feist-Benary synthesis which uses α-halogen ketones and β-dicarbonyl compounds as feedstock. In any event such feedstocks are fossil fuel derived.

In recent years, to reduce the demand for fossil fuels, increased efforts have focused on producing chemicals, including n-butanol, 1,4-BDO and THF, from renewable feedstocks such as sugar-based materials, or their derivatives, including via furan.

A method for obtaining furan from renewable resources involves the decarbonylation of furfural. Examples of reaction processes for achieving this, and the subsequent conversion of the furan into its derivatives, can be found in: (i) Hoydonck, H.E., Van Rhijn, W.M., Van Rhijn, W., De Vos, D.E. & Jacobs, P.A. (2012) "Furfural and Derivatives", in Ulmann's Encyclopedia of Industrial Chemistry (volume 16, pp 285-313), Wiley-VCH Verlag GmbH & Co. KGaA; (ii) Dunlop, A.P. and Peters, F.N., in "The Furans" Reinhold Publ. Co, 1953; (iii) K.J. Zeitsch, in "The Chemistry and Technology of Furfural and its Many By-products" Sugar Series 13, Elsevier, 2000; (iv) Lange, J-P, van der Heide, E, van Buijtenen, J., and Price, R. "Furfural-A Promising Platform for Lignocellulosic Biofuels", ChemSusChem 2012, 5, 150 - 166; and (v) Watson, J.M., Ind. Eng. Chem. Prod. Res. Develop., 1973, 12(4), 310.

Furfural may be obtained from hemicellulose via acid hydrolysis in the liquid phase as well as in the gas phase as described in WO 2002/22593 and WO 2012/041990.

The conversion of furan to THF and 1,4-BDO by hydrogenation in the presence of water, acetic acid and Raney nickel or oxide supported nickel catalyst is described in Watson, J.M., Ind. Eng. Chem. Prod. Res. Develop., 1973, 12(4), 310.

A process for the conversion of furan into THF, 1,4-BDO and n-butanol is taught in US 5905159. This document teaches a process in which furan is converted as a reaction mixture with water and in the presence of hydrogen but in the absence of a water-soluble acid in a single stage over a hydrogenation catalyst. The hydrogenation catalyst of US 5905159 contains at least one element of subgroup I, V, VI, VII or VIII in the form of a compound or in elemental form, with the restriction that the catalyst does not contain nickel alone being applicable. The catalysts taught in US 5905159 generally contain two metals with most containing rhenium as a promoter. The most preferred catalyst taught in US 5905159 for the process is rhenium/ruthenium on active carbon.

US 4476332 describes a process for the production of n-butanol and 1,4-butanediol, comprising contacting furan with hydrogen and water in the presence of a trihaloacetic acid and a catalyst comprising ruthenium on a solid support (non-acidic) such as alumina or aluminosilicate.

Rhenium is a costly metal to be used as a promoter and its avoidance would be desirable.

Further, the production of the ring-opened 1,4-BDO and n-butanol, rather than cyclic THF, has proved challenging when rhenium-free catalysts are used. Known methods in the art provide a mixture of THF, 1,4-BDO and n-butanol. Usually the amounts of THF and 1,4-BDO in such mixtures are greater than the amount of n-butanol.

It would, therefore, be advantageous to provide a method for the production of n-butanol and 1,4-BDO from furan using a simpler and cheaper catalyst than those known in the art. Further, it would be useful to be able to tailor the amount of n-butanol and 1,4-BDO produced, relative to that of THF produced, according to global market conditions for these chemicals. As an example, the size of the n-butanol global market in 2013 was around 3.3 megatons (MT), and those of 1,4-BDO and THF were 1.5 MT and 0.6 MT respectively, but as the production of these chemicals change according to their demand, their price, and hence their profitability, also change. It would, therefore, be desirable for their producers to tailor their production with minimal cost and operation to track the more profitable chemical amongst them.

### Summary of the Invention

Accordingly, the present invention provides a process for the production of n-butanol and 1,4-butanediol, said process comprising contacting furan with hydrogen and water in the presence of a catalytic composition, comprising at least one element selected from those in groups 8, 9, 10 and 11 of the periodic table on a solid support comprising an amorphous or crystalline aluminosilicate in an acidic form, wherein the catalyst does not contain metals selected from those in groups 6 and 7 of the periodic table.

### Detailed Description of the Invention

The present inventors have surprisingly found that solid-supported catalysts comprising at least one element selected from those in groups 8, 9, 10 and 11 of the periodic table supported on an amorphous or crystalline aluminosilicate support in an acidic form can be used as highly active catalysts for the production of n-butanol and 1,4-butanediol in the absence of a promoter metal selected from those in groups 6 and 7 of the periodic table. Further, the composition of the products derived from the hydrogenation of furan can be tailored dependent on the chosen combination of the catalyst support and the catalytically active metal.

More surprisingly, the present inventors have found that the n-butanol:THF and 1,4-BDO:THF product ratios of furan hydrogenation is significantly altered depending on which element of group 8, 9, 10, or 11 is selected as the catalytically active metal in the catalyst used for the hydrogenation reaction.

The at least one element selected from those in groups 8, 9, 10 and 11 of the periodic table is preferably selected from cobalt, nickel, copper, ruthenium, rhodium, palladium, iridium and platinum.

The one or more element selected from groups 8, 9, 10, or 11 may be present on the catalyst in their elemental form or as compounds.

The method of application of the elements of group 8, 9, 10, or 11 the support is not critical and may be effected in a wide range of ways. The elements of group 8, 9, 10, or 11 may be applied to the support using the same or different methods and either sequentially of simultaneously. Suitable methods include, for example, impregnation of the support with solutions or suspensions of the salts, complexes, hydroxides, oxides or other organic or inorganic compounds of the relevant elements, drying and optional calcination. Another possibility for applying the elements of group 8, 9, 10, or 11 to the support is to impregnate the latter with a solution of thermally readily decomposable complexes, for example with carbonyl or hydride complexes of the rhenium and/or palladium, and to heat the carrier thus impregnated to, for example, 150 to 600° C. for thermal decomposition of the absorbed metal compounds. The elements of group 8, 9, 10, or 11 may furthermore be deposited on the catalyst carrier by vapour deposition or by flame spraying. Another preparation method consists of ion-exchange of the support with cationic salts or complexes of the metals followed by drying. Subsequent reduction of the metal compound to the relevant metals or compounds of lower oxidation states by means of a reducing agent may be carried out after any method of deposition.

The total amount of the metal (considered as its elements) on the catalyst may vary within wide ranges, and may be of from 0.01 to 20 wt%, 0.1 to 10 wt% or 0.5 to 5 wt% on the basis of the total weight of the catalyst. Preferably, the total amount of said metal or metals is at least 0.01 wt%, more preferably at least 0.03 wt%, more preferably at least 0.1 wt%, more preferably at least 0.3 wt%, more preferably at least 1.0 wt%, most preferably at least 3.0 wt%. Further, preferably, the total amount of said metal or metals is at most 20 wt%, more preferably at most 15 wt%, most preferably at most 10 wt.

The support in the present invention comprises at least one aluminosilicate in acidic form. Suitable aluminosilicates include acidic forms of both amorphous aluminosilicate and zeolites such as ZSM-5, Beta, Y, Mordenite, ZSM-11 and ZSM-22.

The furan may be contacted with hydrogen either in the gas or the liquid phase.

Suitable conditions for the production of mainly n-butanol and THF from furan include gas- or liquid-phase conditions in the absence or presence of gas or liquid diluent. For liquid phase condition, an inert non-polar or moderately polar solvent, such as a hydrocarbon or oxygenate, can be used. Further conditions include a temperature in the range of from 25 to 250°C, a pressure of from 0.1 to 10MPa and a H₂:furan molar ratio in the range of from 0.2:1 to 100:1, preferably in the range of from 0.2:1 to 10:1 and most preferably in the range from 1:1 to 3:1.

Alternatively, suitable conditions for the production of a mixture of n-butanol, BDO and THF include co-feeding water as a gas or liquid at a water:furan molar ratio in the range of from 0.2:1 to 100:1, preferably in the range of 1:1 to 20:1 and most preferably 3:1 to 10:1. In this embodiment, further suitable conditions include the use of a solvent comprising water and/or oxygenates, preferably the reaction product (THF) or eventually by-products, a temperature in the range of from 100 to 350°C, preferably 120 to 250°C, most preferably 150-200°C, a pressure of from 0.1 to 15MPa, preferably 1-10 MPa and most preferably 3-7 MPa and a H₂:furan molar ratio in the range of from 0.2:1 to 100:1, preferably in the range of from 1:1 to 10:1, most preferably 2:1 to 5:1. In this embodiment, more 1,4-BDO is produced when the process is carried out in liquid water.

The invention will now be illustrated by the following non-limiting examples.

### Examples

A number of catalysts were evaluated in a 16-reactor testing unit that can operate at up to 80 bar and 500°C. The testing unit can be fed with up to 5 gases (hydrogen, CO, N₂, argon and air) and two liquids. The unit allowed for on-line GC analysis of gases and semi-automated offline GC analysis of the liquid product. Gas and liquid product yields were determined in reference to a gas standard (He) and a liquid standard (diethylene-glycol diethyl ether) that were fed together with the gas and liquid feed and were selectively collected in the gas and liquid samples, respectively.

The reactor consisted of SS316 tubes of 4.6 mm ID and 35.5 cm long, of which the central 10 cm length is isothermal. The reactor tubes were loaded with about 1 mL of catalyst, centered in the middle of the reactor while the remaining upper and lower void was filled with inert material such as SiC particles and/or porous SS316 cylinders.

The supports used in Examples 1 to 9 of the invention were either based on amorphous silica-alumina or a mixture of amorphous silica-alumina with H-Beta zeolite. The support indicated as "ASA" contained amorphous silica-alumina, which contained silica and alumina in a weight ratio of 75:25, bound in a weight ratio of 70:30 with alumina (weight ratio of silica-alumina:alumina). The support indicated as "H-Beta/ASA" comprised 4wt% H-Beta zeolite, 66wt% amorphous silica-alumina bound with 30wt% alumina. The support used in examples 5-6 and indicated as "H-Beta" comprised 35 w% of zeolite Beta that has a silica/alumina weight ratio of 730, is in acidic form and bound with alumina. The supports used in Examples 10 to 13 indicated as "H-ZSM5" comprised a silica bound ZSM-5 zeolite in its acidic form with a zeolite content of 30-60 w% and with an silica:alumina weight ratio 30-50 in the zeolite.

These catalysts were prepared by incipient wetness impregnation of the support with solution of the following salts: Pt(NH₃)₄(N0₃)₂, Ru(NO₃)₃NO. The solutions were prepared with the concentration required to achieve the targeted metal loading. The catalysts were dried at 120°c for 2 h in air and for half an hour at 225°C temperature.

The catalysts were dried and reduced for 1 h at 75°C, 4 h at 120°C and 4 h at 275°C under a 30% H₂/70% N₂ flow of 0.625 NL/h (per reactor) at nearly atmospheric pressure. Subsequently, the temperature is lowered to 120°C, the pressure is raised to 50 atmosphere and the gas flow set to 0.25 NL/h (per reactor) and 100% H₂ and subsequently 0.25 NL/h and 85% H₂/15% H₂ to be ready for start-up.

The gas feed consisted of a mixture of 10% He and 90% H₂ and was fed at a rate of about 280 NL per liter catalyst bed per hour. The liquid feed consisted of a mixture of 24 w% furan, 21 w% water, 50 w% ethanol and 4 w% standard. The liquid feed was introduced at a rate of about 0.8 litre per litre catalyst bed per hour. The run was carried out at a pressure of 50 bars. The temperature was ramped from 140 to 200°C by steps of 20°C and back to 160°C. The run lasted for 200-250 hours in total.

The average yields measured are reported in Table 1. The yields are expressed as fraction of the carbon of furan that is converted into the desired product. The yield may occasionally add up to slightly more than 100C% as results of experimental inaccuracies.

As shown in Table 1, good overall yields were obtained for the process of the invention, with the combined yields of butanol and 1,4-BDO being higher than that of THF.

Advantageously, it was also found that using different metals in the process of the invention allows said process to be tailored to provide higher relative yields of either 1,4-BDO or n-butanol.

**Table 1 - Catalysts of the Invention**

| | **Support** | **Metal** | **Metal w%** | **Temp °C** | **BDO yield C%** | **THF yield C%** | **NBA yield C%** |
|---|---|---|---|---|---|---|---|
| **1** | H-Beta/ASA | Pt | 0.8 | 160 | 6.5 | 18.5 | 31.4 |
| **2** | H-Beta/ASA | Pt | 0.8 | 185 | 4.2 | 16.3 | 23.6 |
| **3** | ASA | Pt | 0.5 | 160 | 8.7 | 19.1 | 49.1 |
| **4** | ASA | Pt | 0.5 | 185 | 6.8 | 19.0 | 48.7 |
| **5** | H-Beta | Ru | 0.5 | 160 | 26.2 | 35.5 | 14.7 |
| **6** | H-Beta | Ru | 0.5 | 185 | 17.3 | 36.2 | 16.2 |
| **7** | ASA | Ru | 0.1 | 120 | 7.3 | 31.5 | 5.2 |
| **8** | ASA | Ru | 0.1 | 140 | 0.6 | 3.3 | 0.9 |
| **9** | ASA | Cu | 15% | 140 | 0.7 | 3.9 | 1.3 |
| **10** | H-ZSM5 | Pt | 1.0 | 140 | 1.5 | 4.2 | 6.3 |
| **11** | H-ZSM5 | Pt | 1.0 | 160 | 1.3 | 6.3 | 10.2 |
| **12** | H-ZSM5 | Pt | 1.0 | 180 | 2.0 | 5.7 | 14.3 |
| **13** | H-ZSM5 | Pt | 1.0 | 200 | 2.1 | 6.4 | 20.9 |

Comparative catalysts were produced and tested according to similar processes. The results of these tests are shown in Table 2. It can be seen that catalysts containing rhenium provide much higher levels of THF rather than 1,4-BDO and/or n-butanol. Catalyst 26, which does not contain rhenium, and also does not use an acidic aluminosilicate support provides very low levels of 1,4-BDO, with the vast majority of the product being THF.

**Table 2 - Comparative Examples**

| | **Support** | **Metal 1** | **Metal 2** | **Metal 1 w%** | **Metal 2 w%** | **THF Yield C%** | **BDO Yield C%** | **NBA Yield C%** |
|---|---|---|---|---|---|---|---|---|
| **14** | Carbon | Pt | Re | 0.50 | 5.0 | 63.9 | 12.2 | 12.7 |
| **15** | TiO₂ | Pt | Re | 0.10 | 5.0 | 48.9 | 4.2 | 20.5 |
| **16** | ZrO₂ | Pt | Re | 0.10 | 5.0 | 48.0 | 1.7 | 18.8 |
| **17** | ZrO₂ | Pt | Re | 0.50 | 3.0 | 49.8 | 0.8 | 17.3 |
| **18** | Carbon | Ru | Re | 1.00 | 5.0 | 66.0 | 12.1 | 15.6 |
| **19** | Carbon | Ru | Re | 0.20 | 2.0 | 3.3 | 3.7 | 3.3 |
| **20** | TiO₂ | Ru | Re | 0.10 | 10 | 24.4 | 3.0 | 10.3 |
| **21** | TiO₂ | Ru | Re | 1.00 | 10 | 59.1 | 1.7 | 26.9 |
| **22** | TiO₂ | Ru | Re | 0.50 | 5.0 | 58.1 | 1.3 | 22.8 |
| **23** | TiO₂ | Ru | Re | 1.67 | 5.0 | 48.3 | 0.1 | 10.5 |
| **24** | Carbon | Ru | Ni | 0.52 | 4.0 | 87.9 | 5.1 | 2.2 |
| **25** | Carbon | Ru | Cu | 3.00 | 3.0 | 35.3 | 1.8 | 16.1 |
| **26** | Ce/Zr* | Ru | - | 1.5 | - | 57.7 | 0.1 | 13.0 |

| | | | | | | | | |
|---|---|---|---|---|---|---|---|---|
| * Ce_{0.75}Zr_{0.25}O₂ | | | | | | | | |

## Claims

1. A process for the production of n-butanol and 1,4-butanediol, said process comprising contacting furan with hydrogen and water in the presence of a catalytic composition, comprising at least one element selected from those in groups 8, 9, 10 and 11 of the periodic table on a solid support comprising an amorphous or crystalline aluminosilicate in an acidic form, wherein the catalyst does not contain metals selected from those in groups 6 and 7 of the periodic table.

2. A process according to claim 1, wherein the solid support comprises an amorphous aluminosilicate in an acidic form.

3. A process according to claim 1 or claim 2, wherein the solid support comprises a zeolite in an acidic form.

4. A process according to claims 1 to 3, wherein at least one element selected from those in groups 8, 9, 10 and 11 of the periodic table is selected from the group of at least one of cobalt, nickel, copper, ruthenium, rhodium, palladium, iridium or platinum in their elemental form or as compounds.

5. A process according to any one of claims 1 to 4, wherein the total amount of the at least one element selected from those in groups 8, 9, 10 and 11 of the periodic table considered as its element on the catalyst is in the range of from 0.01 to 20wt%.

6. A process according to any one of claims 1 to 5, wherein the furan is contacted with hydrogen in the liquid phase at a temperature in the range of from 25 to 250°C, a pressure of from 0.1 to 10MPa and a H₂:furan molar ratio in the range of from 0.2:1 to 100:1.

7. A process according to any one of claims 1 to 5, wherein the furan is contacted with hydrogen and water is co-fed at a water:furan molar ratio in the range of from 0.2:1 to 100:1, at a temperature in the range of from 100 to 350°C, a pressure of from 0.1 to 15MPa and a H₂:furan molar ratio in the range of from 0.2:1 to 100:1.

## Patentansprüche

1. Vorgang zum Herstellen von n-Butanol und 1,4-Butandiol, wobei der Vorgang das Kontaktieren von Furan mit Wasserstoff und Wasser in Gegenwart einer katalytischen Zusammensetzung, die mindestens ein Element umfasst, das aus denen in den Gruppen 8, 9, 10 und 11 des Periodensystems ausgewählt ist, auf einem festen Träger, umfassend ein amorphes oder kristallines Aluminosilikat in einer sauren Form, umfasst, wobei der Katalysator keine Metalle enthält, die aus denen in den Gruppen 6 und 7 des Periodensystems ausgewählt sind.

2. Vorgang nach Anspruch 1, wobei der feste Träger ein amorphes Aluminosilikat in einer sauren Form umfasst.

3. Vorgang nach Anspruch 1 oder Anspruch 2, wobei der feste Träger einen Zeolith in einer sauren Form umfasst.

4. Vorgang nach den Ansprüchen 1 bis 3, wobei mindestens ein Element, ausgewählt aus denen in den Gruppen 8, 9, 10 und 11 des Periodensystems, aus der Gruppe mindestens eines der Elemente Cobalt, Nickel, Kupfer, Ruthenium, Rhodium, Palladium, Iridium oder Platin in ihrer elementaren Form oder als Verbindungen ausgewählt ist.

5. Vorgang nach einem der Ansprüche 1 bis 4, wobei die Gesamtmenge des mindestens einen Elements, ausgewählt aus denen in den Gruppen 8, 9, 10 und 11 des Periodensystems, betrachtet als sein Element auf dem Katalysator, im Bereich von 0,01 bis 20 Gew.-% liegt.

6. Vorgang nach einem der Ansprüche 1 bis 5, wobei das Furan mit Wasserstoff in der flüssigen Phase bei einer Temperatur im Bereich von 25 bis 250 °C, einem Druck von 0,1 bis 10 MPa und einem H₂:Furan-Molverhältnis im Bereich von 0,2:1 bis 100:1 kontaktiert wird.

7. Vorgang nach einem der Ansprüche 1 bis 5, wobei das Furan mit Wasserstoff in Kontakt gebracht wird und Wasser bei einem Wasser:Furan-Molverhältnis im Bereich von 0,2:1 bis 100:1 bei einer Temperatur im Bereich von 100 bis 350 °C, einem Druck von 0,1 bis 15 MPa und einem H₂:Furan-Molverhältnis im Bereich von 0,2:1 bis 100:1 gleichzeitig eingespeist wird.

## Revendications

1. Procédé pour la production de n-butanol et 1,4-butanediol, ledit procédé comprenant la mise en contact du furane avec de l'hydrogène et de l'eau en présence d'une composition catalytique, comprenant au moins un élément choisi parmi ceux des groupes 8, 9, 10 et 11 du tableau périodique, sur un support solide comprenant un aluminosilicate amorphe ou cristallin sous forme acide, le catalyseur ne contenant pas de métaux choisis parmi ceux des groupes 6 et 7 du tableau périodique.

2. Procédé selon la revendication 1, dans lequel le support solide comprend un alumonisilicate amorphe sous forme acide.

3. Procédé selon la revendication 1 ou la revendication 2, dans lequel le support solide comprend une zéolite sous forme acide.

4. Procédé selon les revendications 1 à 3, dans lequel au moins un élément choisi parmi ceux des groupes 8, 9, 10 et 11 du tableau périodique est choisi dans le groupe constitué d'au moins un élément parmi le cobalt, le nickel, le cuivre, le ruthénium, le rhodium, le palladium, l'iridium ou le platine sous leur forme élémentaire ou en tant que composés.

5. Procédé selon l'une quelconque des revendications 1 à 4, dans lequel la quantité totale de l'au moins un élément choisi parmi ceux des groupes 8, 9, 10 et 11 du tableau périodique considéré comme son élément sur le catalyseur se situe dans une plage allant de 0,01 à 20 % en poids.

6. Procédé selon l'une quelconque des revendications 1 à 5, dans lequel le furane est mis en contact avec l'hydrogène dans la phase liquide à une température comprise dans une plage allant de 25 à 250°C, à une pression allant de 0,1 à 10 MPa et à un rapport molaire H₂:furane compris dans une plage allant de 0,2:1 à 100:1.

7. Procédé selon l'une quelconque des revendications 1 à 5, dans lequel le furane est mis en contact avec de l'hydrogène et de l'eau, et de l'eau est alimenté conjointement à un rapport molaire eau:furane compris dans une plage allant de 0,2:1 à 100:1, à une température comprise dans une plage allant de 100 à 350°C, à une pression allant de 0,1 à 15 MPa et à un rapport molaire H₂:furane compris dans une plage allant de 0,2:1 à 100:1.
